# EUROPEAN PATENT SPECIFICATION

(11) **EP 3 035 996 B1**
(45) Date of publication and mention of the grant of the patent: **31.10.2018**
(21) Application number: 14853048.8
(22) Date of filing: 25.09.2014
(51) Int. Cl.: A61M 39/02, A61M 25/01, A61M 37/00

(54) **INDICIA BEARING SEPTUMS, METHODS OF MANUFACTURING THE SEPTUMS AND PORTAL THEREFOR**
MARKIERUNGSZEICHENTRAGENDE SEPTEN, VERFAHREN ZUR HERSTELLUNG DER SEPTEN UND PORTAL DAFÜR
SEPTUMS PORTEURS D'ÉLÉMENTS VISUELS, PROCÉDÉS DE FABRICATION DESDITS SEPTUMS ET PORTE ASSOCIÉE

(30) Priority: 08.10.2013 US 201361888047 P
(43) Date of publication of application: 29.06.2016
(60) Divisional application: 18192180.0
(73) Proprietor: Smiths Medical ASD, Inc., Plymouth MN 55442 (US)
(72) Inventor: FINBERG, Kristin, Minneapolis, MN 55410 (US); HOOPLE, Cal Aaron, Hudson, WI 54016 (US); KUBAS, Amy, Hugo, MN 55038 (US); TRAVIS, Ronald Gene, Spring Lake Park, MN 55432 (US); WOO, Louis, Alexandria, VA 22314 (US)
(74) Representative: Hedges, Martin Nicholas
(86) International application number: PCT/US2014/057327
(87) International publication number: WO 2015/053944

(56) References cited:
- WO-A2-2013/138813
- US-A1- 2011 257 609
- US-A1- 2011 288 503
- US-A1- 2012 059 250
- US-A1- 2012 078 202
- US-A2- 2010 211 026
- US-B1- 6 287 293

## Description

### Field of the Invention

The instant invention relates to implantable medical devices and more particularly to portals having an indicia bearing septum that can readily be identified after being implanted into a patient, and methods of manufacturing the indicia bearing septum.

### Background of the Invention

A port, or portal, is a medical device having a housing fitted with a resealable septum to provide a reservoir that is implantable subcutaneously in a patient so that the fluid stored in the reservoir may be directed, by means of a catheter attached to the output of the portal, to a particular location in a" patient. To gain access to the reservoir, a cannula is inserted through the resealable elastomeric septum so that fluid may be input to or withdrawn from the reservoir.

US patent No. 8,092,435, assigned to the same assignee as the instant application, discloses a portal that has a septum embedded indicia. The septum has a base onto which a depression is formed on the top surface thereof, so that radiopaque material may be injected into the depression. A silicone layer then covers the top of the septum base to seal-in the hardened radiopaque material. That the radiopaque material needs to be injected in fluid form into the septum base is a time consuming process. The instant invention provides an improved indicia bearing septum(s) and a method(s) of manufacturing such indicia bearing septum with substantial time savings and improved efficiencies.

### Brief Summary of the Invention

US-A-6287293 discloses a method and apparatus for locating the injection point of an implanted medical device comprising locating apparatus in the form of radiopague rings made of a metallic substance, which rings are spaced apart at different heights on an implanted medical device, a stereoscopic effect resulting from fluoroscopically viewing the device along an axis not parallel to the axis along which the rings are spaced, thereby allowing readily determination of the orientation of the device so as to prevent accidental injection of drugs into the hard backside of the drug port.

Other prior art is disclosed in WO2013/138813, US2012/078202 and US2011/257609.

### Brief Summary of the Invention

To shorten the time and increase the efficiency in the manufacturing thereof, the indicia bearing septum of the instant invention is manufactured by mounting into the cavity formed by the depression at the top surface of the septum base an insert that has the same given configuration as the impression provided by the depression. The insert is a solid one-piece radiopaque material that is formed from a mold in a batch of multiple inserts. Each of the multiple inserts retrieved or extracted from the mold is mounted to a corresponding septum base, with the insert mounted septum base being thereafter bondedly covered by a silicone layer. Thus, instead of injecting fluidized radiopaque material individually to the depression formed at each of the septums, and waiting for the radiopaque material to cure and harden, the instant invention manufacturing process eliminates the need to inject fluid to each of the septums and the need to wait for the fluidized radiopaque material to cure correctly and harden before applying the silicone layer. As a result, operationally, the inventive manufacturing process saves time and increases the efficiency of manufacturing the indicia bearing septums. Moreover, by molding the radiopaque indicia, due to the compacting of the radiopaque material during the molding process, the resulting insert mounted to the septum base, when viewed under radiographic imaging, provides an improved visual representation than the indicia embedded septums disclosed in the aforenoted '435 patent.

An alternative method of manufacturing an indicia bearing septum of the instant invention is to provide a once-piece combination insert and top layer that has impregnated therein a radiopaque material. The radiopaque impregnated insert has a leg formation that has the same configuration as the depression formed at the top surface of the septum base. The depression at the septum base forms a cavity that has a sufficient depth so that when the leg formation from the combination insert is fully mounted thereinto, a viewable information bearing indicia is provided by the leg formation under radiographic imaging. When properly bonded to the septum base, the septum thus formed from the alternative manufacturing process has a top that is opaque visually, and yet provides an information bearing indicia when viewed under x-ray or computer tomography imaging.

With both of the afore-discussed methods for manufacturing the inventive septum, there is no longer any need to wait for the liquid radiopaque material injected into the depression at the septum base to solidify, harden and cure. As a result, the inventive manufacturing processes are able to produce information bearing septums, and of course the ports into which the septums are sealingly fitted, in greater numbers to the tune of producing a minimum of 20-30 times more septums per hour than septums that are produced in accordance with the method disclosed in the afore-noted '435 patent. Further, the quality of the septums thus manufactured is higher than the previous generation of septums since controls that were required to be in place to ensure that the fluid radiopaque material cures properly are no longer needed. Furthermore, the molded insert provides a better viewing of the indicia under radiographic imaging due to the compacting of the radiopaque material during the molding process.

Accordingly, a first aspect of the present invention provides a method of manufacturing an indicia bearing septum according to claim 1.

The present invention further provides a port implantable into a patient according to claim 8.

The indicia at the septum provides at least an identification of the port under x-ray or computer tomography imaging. Moreover, the indicia at the septum provides a location whereby the clinician can access the port by using a cannula to pierce through the resealable septum at that location.

### Brief Description of the Figures

The present invention will become apparent and the invention itself will be best understood with reference to the following description of the present invention taken in conjunction with the accompanying drawings, wherein:
Fig. 1 is a disassembled view of the subcutaneous implantable portal of the instant invention;
Fig. 2 is a perspective view of the septum of the instant invention that is a part of the portal shown in Fig. 1;
Fig. 3 is a plan view of the portal of Fig. 1 ;
Fig. 4 provides a perspective view of an assembled portal of the instant invention;
Fig. 5 is a disassembled view of the septum of the instant invention;
Fig. 6A is a perspective view of the assembled septum of the instant invention;
Fig. 6B is a plan view of the inventive septum of Fig. 6A;
Fig. 6C is a cross-sectional view of the inventive septum;
Fig. 7 illustrates the mold for manufacturing the radiopaque insert for the inventive septum;
Fig. 8 illustrates the mold for making of the septum base of the inventive septum;
Fig. 9 is an illustration of an alternative embodiment of the inventive septum; and
Fig. 10 is a cross-sectional view of the inventive septum shown in Fig. 9.

### Detailed Description of the Invention

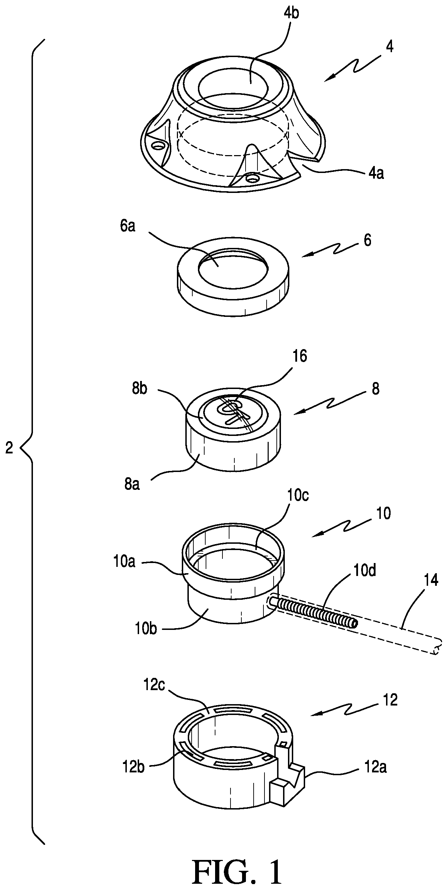

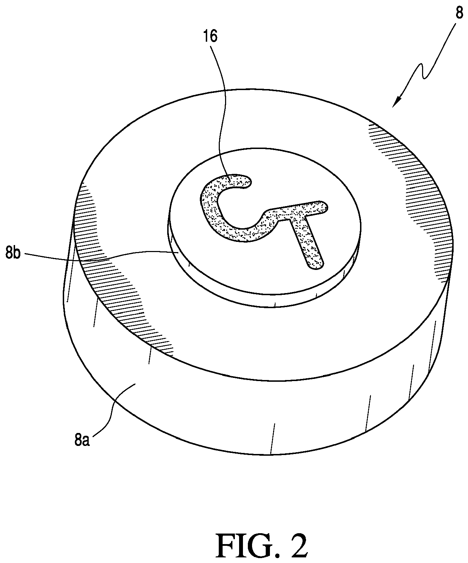

Fig. 1 is similar to Fig. 2 of the above-discussed US patent No. 8,092,435, the disclosure of which is incorporated by reference herein. The only difference is the septum for the portal or port 2 shown in the disassembled view. In brief, the subcutaneous implantable port 2 enables the port to be located once it is subcutaneously implanted into the patient, so that the fluid, for example a liquid medicament input and stored in the reservoir of the port for example by means of a pump or a syringe, can be output to a particular destination inside the patient. Alternatively, fluid in the reservoir may be withdrawn by using a syringe, for example. The port of the instant invention provides an identification of the port and the location of the port inside the patient, under radiographic imaging including x-ray and computer tomography imaging.

Fig. 1 shows a disassembled view of the various components or elements of the port of the instant invention. In particular, port 2 includes a housing 4, a cap 6, a septum 8, a reservoir body 10 and a housing base 12. Reservoir body 10 is cup-shaped and is shown to have an upper portion 10a and a lower portion 10b, which includes the base of the reservoir body 10. A shoulder 10c joins upper portion 10a to lower portion 10b. An outlet 10d extends from lower portion 10b of reservoir body 10. A conduit or catheter 14, in phantom line, is connected to outlet 10d for transporting the fluid stored in reservoir body 10 to a selected location within the patient, when port 2 is implanted subcutaneously in the patient.

Fitted to the upper portion 10a of reservoir body 10, with shoulder 10c providing a rest stop therefor, is septum 8. As shown in Fig. 1, septum 8 is a one-piece integral unitary component that has a septum base 8a and an elevated top 8b. In fact, septum 8 is made in multiple steps that will be described in detail, *infra.* An information bearing indicia element or insert 16 is embedded in septum 8. The process of embedding insert 16 in septum 8 will also be described in detail, *infra.*

Once fitted to upper portion 10a of reservoir body 10, to hold septum 8 in place, cap 6 is friction fitted over top portion 10a of reservoir body 10. For the exemplar port shown, both cap 6 and reservoir body 10 are made from plastic material, titanium or some other inert metal acceptable for implantation to a patient. An opening 6a at cap 6 exposes top layer 8b, and more particularly the information bearing indicia 16 embedded in septum 8. The assembled reservoir housing -- made up of reservoir body 10, septum 8 and cap 6 -- is then placed in housing base 12, which is in the form of a collar with its inside diameter having a dimension sufficient to receive reservoir body 10. A notched support 12a at a side of housing base 12 provides support to outlet 10d. Housing 4 then is positioned over housing base 12 to envelope the assembled reservoir housing. A slot 4a at the lower portion of housing 4 provides accommodation for outlet 10d extending out from reservoir body 10. A top opening 4b at housing 4 exposes the top surface of septum 8, and therefore the information bearing indicia embedded in septum 8. To prevent separation, housing 4 and housing base 12 are ultrasound welded, possibly at the location defined by grooves 12b at the lip 12c of housing base 12. Housing 4 and housing base 12 may be made from conventional medical plastics material to reduce the cost and the weight of the port.

Fig. 2 is a perspective view of septum 8 of the instant invention. Note that the information bearing indicia 16 is a one-piece insert that is embedded into the top surface and the upper portion of septum base 8a. The slightly upraised layer 8b of septum 8 may be considered to the upper portion of the septum. The finished exemplar septum 8 in Fig. 2, although not clearly shown, has a clear silicone layer formed over the top of the septum base to encapsulate and/or insulate the information bearing indicia insert 16 and the top of the septum from the environment. As shown, indicia 16 is a one continuous insert element that has a given configuration, shown in the exemplar septum 8 of Fig. 2 to be a conjoint "C" and "T". It should be appreciated however that other configurations may also be used as indicia 16, including symbols and non-alphanumeric characters. So, too, instead of one single continuous configuration, indicia 16 may be formed by multiple inserts that are separately provided in and viewable from septum 8.

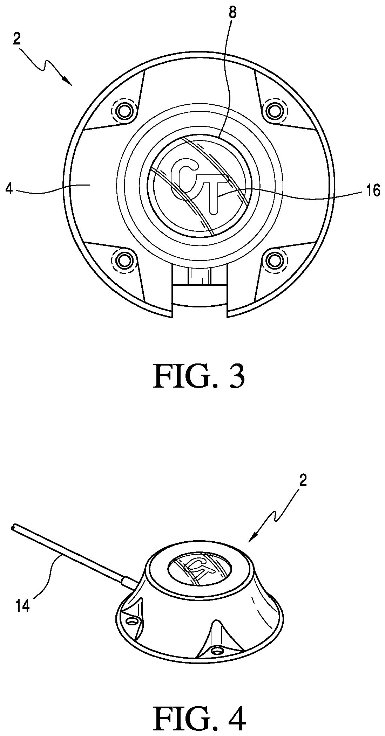

Fig. 3 is a plan view of the assembled port 2. Fig. 4 shows a perspective view of the assembled port 2 having a catheter 14 connected to its outlet.

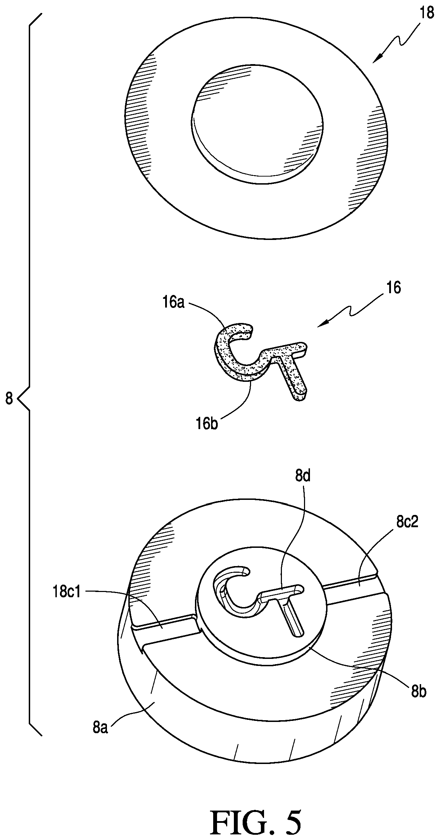

With reference to Fig. 5, a disassembled exemplar septum 8 of the instant invention is shown to have a septum base or puck 8a that has a raised layer 8b. Septum base 8a may be made from a silicone gum stock or LIM (liquid injection molding) material, for example. Base 8a is fabricated to have a thickness in a cross section to enable at least the lower portion of it to be form fitted into the upper portion 10a of reservoir housing 10. The bottom surface of septum base 8a may be flat. Two channels 8c1 and 8c2 are provided for the inflow and outflow, respectively, of liquid silicone to form the clear silicone layer, designated 18, that covers the top of the septum base to complete the manufacturing of the septum. As noted above, septum base 8a has a raised layer 8b where a particular impression, mark or indicia 8d of a given configuration is formed as a depression 8d. As shown, depression 8d is a continuous trench, groove or cavity that provides a readable information bearing indicia to a viewer. In the exemplar septum base 8a shown in Fig. 5, depression 8d is in the form of an alphanumeric information bearing indicia cavity that has combined and joined the letters "C" and "T". That the depression 8b is formed as one continuous trench, groove or cavity provides for easy assembly for the septum, as will be described below.

As disclosed in the aforenoted '435 patent, for the previous generation of septum, a liquid radiopaque material such as barium sulfate (BaSo4), or some other similar radiopaque material viewable under radiographic imaging, is injected into the depression 8d by using an injection mechanism such as a syringe. After the liquid radiopaque material is injected onto the septum, it has to be solidified and hardened, and cured, before anything else can be done. As a result, a waiting time is incurred in the manufacturing of the previous generation septum.

The septum of the instant invention eliminates this waiting time by providing a one-piece solid radiopaque insert 16 that fittingly mounts into the cavity formed by depression 8d in septum base 8a. As shown in Fig. 5, insert 16 has the same configuration as that formed by depression 8d on the top surface of septum base 8a. For ease of handling, and also to avoid confusion, the manufacturing process may be such that the cavity effected by depression 8d at septum base 8a has a curved internal trench surface, i.e., a groove. On the other hand, insert 16 is molded to have a flat top surface 16a but an outwardly curved under surface 16b so that insert 15 may be fittingly mounted into the cavity formed by depression 8d. After insert 16 is mounted into depression 8d, the top surface of septum base 8a, which may be flush with the top surface of the insert, is coated with a liquid silicone layer, identified as top layer 18, so that the radiopaque insert 16 and the top of the septum are encapsulated and therefore isolated from the environment.

With the embedding of the one piece solid insert 16 into the septum base 8a, since the radiopaque material is white whereas the septum base formed from silicone is transparent or translucent as is the silicone top layer 18, the clinician can readily visually view the indicia formed by insert 16 from septum 8. Moreover, the port described above manufactured with the exemplar septum shown in Fig. 5, when implanted to a patient, would provide a readable indicia under x-ray or computer tomography. It should moreover be understood that the readable indicia can be read to represent certain characteristics of the port, including for example whether the port is adapted to be used for power injection and the storage capacity of the port fluid reservoir.

As discussed above, for the prior generation septum manufactured under the '435 patent, a liquid radiopaque material has to be injected into the depression formed at the septum base. The process of injecting the liquid radiopaque material is tedious, takes a substantial amount of time and requires a great amount of quality control be in place to ensure that the liquid radiopaque material properly solidifies and cures. Moreover, blemishes or defects formed in the liquid injection radiopaque material is something that is not readily detectable. With the instant invention where a one-piece solid insert is directly mounted into the cavity formed by the depression at the septum base, a number of advantages are achieved. Foremost, the increase in manufacturing efficiency that results from the molding of a great number of inserts, so that the number of septums produced per hour under the manufacturing process of the instant invention increases at least by 20 to 30 fold. Further, there is an improvement in the quality control insofar as blemishes in an insert are readily detected when the insert is extracted from its mold. This ensures that the radiopaque insert is fully cured and is ready to be inserted into a corresponding septum base. Furthermore, there is a great deal of cost savings with the manufacturing of a one-piece solid insert due to the fact that the inserts are produced by molding, and each insert mold can contain multiple numbers of the inserts. Furthermore, that the radiopaque insert is formed by molding means that the liquid radiopaque material injected into the insert mold is compacted during the molding process to thereby provide a tightly compacted solid insert, which in turn provides a better and more clear view of the insert under radiographic imaging.

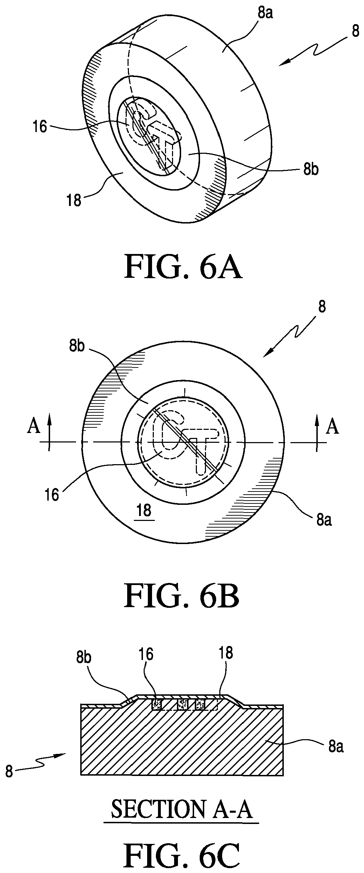

Fig. 6A shows an assembled inventive septum in a perspective view with the embedded radiopaque insert shown in dotted lines. Fig. 6B shows a plan view of inventive septum 8, and Fig. 6C is a cross-sectional view along section A--A of septum 8.

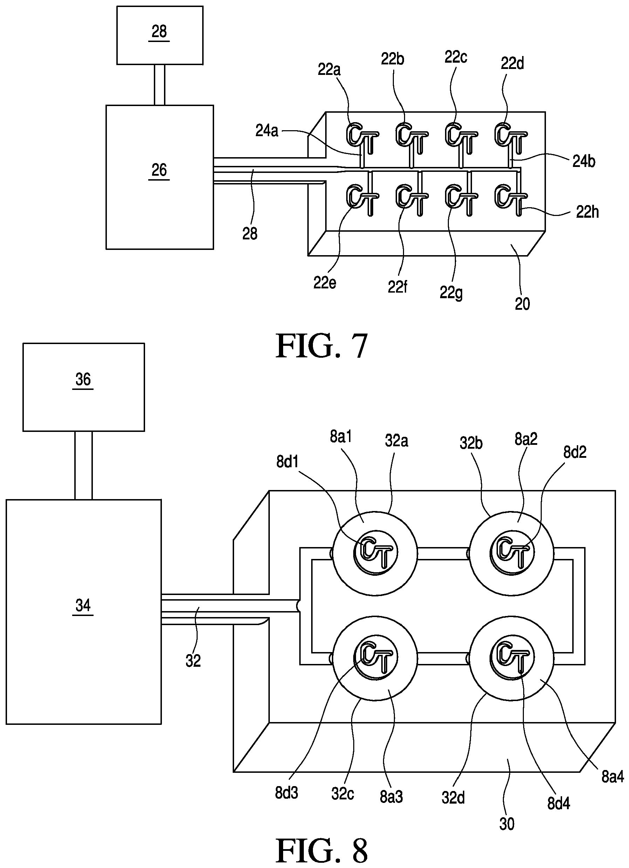

An exemplar process of manufacturing the radiopaque insert for the septum of the instant invention is shown in Fig. 7. As shown, an insert mold 20 is made to have a plurality of molding cavities, for example the eight cavities shown in the exemplar mold 20. There could be a smaller or a greater number of molding cavities 22 than that shown in Fig. 7. Each of the molding cavities 22 is formed in the shape of the given configuration of the insert 16, in this example the conjoint "CT" configuration, that corresponds to the configuration of the depressed impression formed on the septum base to which the insert is to be mounted. A fluid flow channel 24 connects mold 20 to a pump 26 that feeds a liquid radiopaque material, for example barium sulfate (BaSO4), from a liquid radiopaque material store 28 to mold 20. Channel 24 delivers the liquid radiographic material to its different branches, of which only branches 24a and 24b are labeled, so that the liquid radiopaque material fills each of the molding cavities 22a-22h. Mold 20 has a cover (not shown for sake of clarity) that covers mold 20 so that the liquid barium is injected into the different molding cavities and is compacted separately in each of the cavities, as is conventionally known in injection molding. With the cover removed, and the liquid radiopaque material having been solidified and hardened, the thus cured multiple one-piece solid inserts 22a-22h may be extracted or retrieved from the respective molding cavities of mold 20. Each of the extracted inserts is then mounted to the cavity that is formed by the depression at a corresponding septum base, for example 8d at septum base 8a, as described previously. Thus, with the manufacturing process as discussed above, a large number of radiopaque inserts may be molded for each batch of molding.

Fig. 8 shows a septum mold 30 wherein a plurality of septum cavities, for example the four cavities 32a-32d, are shown. For the sake of convenience, Fig. 8 shows only the base of the mold, with the top of the mold having been removed, and the four septum bases formed in the mold, so that the respective impressions formed by the corresponding depressions 8d are shown for the molded septum bases. For clarification purposes, the septum bases in Fig. 8 are labeled 8a1 -8a4, with their respective depressions labeled 8d1 - 8d4. The thus formed septum bases may be extracted from the mold. Each of the septum bases then would have a corresponding radiopaque insert 16 mounted into the cavity formed by the depression at the top surface and the upper portion of the septum base. The liquid silicone stock to form the septum bases is injected to mold 30 by way of flow channel 32, with a pump 34 providing the liquid silicone from a liquid silicone store 36.

As discussed above, after a radiopaque insert having the same configuration as the impression created by the depression formed in the septum base is inserted into the cavity formed by the depression, a liquid layer of silicone is injected or placed over the top of the septum base, so that a clear silicone layer is bonded to the septum base to form the inventive septum. As was pointed out above, the respective top surfaces of the insert and the septum base may be flush with each other to ensure that the clear silicone layer covers both top surfaces to thereby provide an evenly distributed insulation layer.

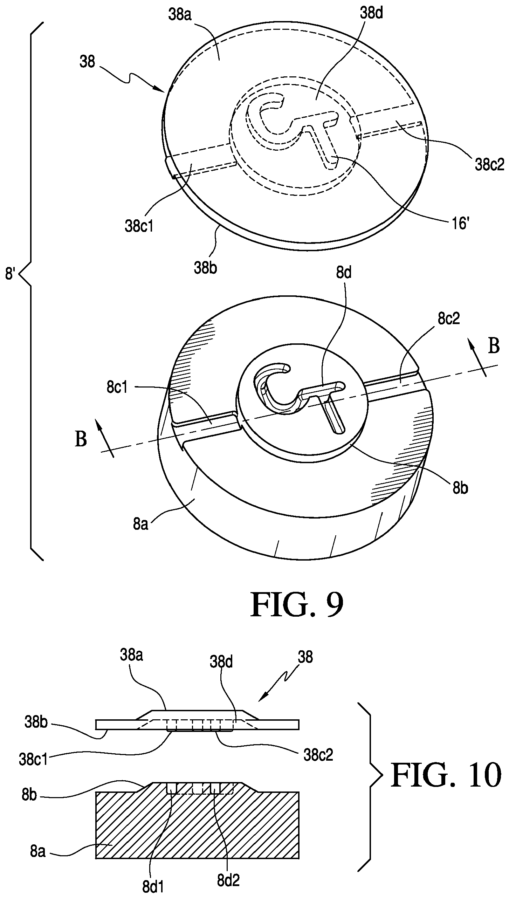

An alternative process for manufacturing the information bearing indicia embedded septum of the instant invention is illustrated in Figs. 9 and 10. For this alternative embodiment, the septum base is made in identical fashion as discussed in the earlier embodiment. Thus, the same reference numbers are used to identify the different parts of the septum base.

In the alternative embodiment, instead of a solid insert, a top layer with an integral insert is formed from a mold to have a configuration that form fits over the top of septum base 8a. Septum top 38 may be formed from a liquid silicone impregnated with a radiopaque material, for example barium sulfate (BaSO4), so that the complete top layer is radiopaque. As shown, top layer 38 has an upper or top surface 38a and a lower or bottom surface 38b that has hanging downwardly therefrom a number of legs or extensions that together form a downwardly extending leg formation or insert that has the same configuration as insert 16 shown in Fig. 5, for example the exemplar conjoint "CT" configuration. This downward extension or leg formation is labeled 16' in the septum top layer 38 shown in Fig. 9. There may also be two integral arms 38c1 and 38c2, shown in dotted lines, that hang downwardly from the bottom surface 38b of top layer 38. Downwardly extending arms 38c1 and 38c2 would fittingly mate to channels 8c1 and 8c2, respectively, formed at the top of septum base 8a. There is moreover a slightly concave cavity 38d, also shown in dotted line, that enables septum top 38 to formingly fit over the raised layer 8b at septum base 8a. For the exemplar septum 8' shown in Fig. 9, there is therefore only two components, i.e., the one-piece solid radiopaque septum top 38 form fitted to the top of septum base 8a. Once positioned and fitted onto septum base 8a, septum top 38 may be bonded to septum base 8a by a number of conventional known methods such as for example ultrasound bonding or gluing.

Fig. 10 shows a cross-sectional view B --B of the septum base 8a and the septum top 38 superposed thereover. As shown, legs 38c1 and 38c2 extending downwardly from the bottom surface 38b of septum cover 38 are the portions of the downward extending insert 16' that mount into the cavities 8d1 and 8d2 shown for the cross-sectional view of septum base 8a. For the septum base embodiment shown in Figs. 9 and 10, the depth of the depression 8d may be deeper than the depression for the embodiment shown in Fig. 5, so that once septum top 38 is bonded to septum base 8a, with the downward hanging inserts 16' fitted into the cavity formed by the depression 8d, the greater length of the insert 16' means that that portion of the radiopaque material can more readily be seen, and be differentiated from the rest of top layer 38, when the septum is view under radiographic imaging, even though septum top 38 is radiopaque.

There are advantages for utilizing the alternative embodiment of the one piece combination top and leg formation layer shown in Figs. 9 and 10. One advantage is that since septum top 10 is impregnated with radiopaque material, the top of the septum formed therewith would be just like other ports except that it is cloudy or white, depending on the radiopaque material used. That notwithstanding, a readable indicia nonetheless can be viewed under radiographic imaging. Further, with the alternative embodiment, the manufacturing of an information bearing indicia septum becomes easier insofar as both the septum base and the septum top that form the inventive indicia embedded septum are separately molded and put together in one single step, therefore resulting in greater efficiency and decreased cost. As is the case with the septum shown in Fig. 5, the septum shown in Figs. 9 and 10 is fitted into reservoir housing 10 shown in Fig. 1, during the assembly of the port 2 of the instant invention.

In place of the top layer insert separately formed at a different mold in the manner described above in Fig. 7, the combination top layer for the septum of the embodiment shown in Figs. 9-10 may be formed directly from the same mold that forms the septum base. For this manufacturing process, the top portion of the mold that covers the septum base has a cavity facing the cavity at the septum base portion of the mold so that the liquid silicone impregnated with the radiopaque material may be injected into the mold, and flow via the respective input channel 8c1 and out flow channel 8c2, after the formation of the septum bases. The injected liquid silicone would fill the impression cavity formed by depression 8d and bonds to the top surface of the septum base to form the one piece combination top and leg formation insert thereat. After the liquid top layer is cured and hardened, the thus formed one piece inventive septum may be removed from the mold.

## Claims

1. A method of manufacturing an indicia bearing septum, comprising the steps of:
(a) providing an elastomeric septum (8);
(b) forming a cavity at an upper portion (8b) of the septum (8) by at least one depression (8d) of a given configuration into a top surface of the septum (8); and **characterized by** the further steps of:
(c) molding a radiopaque insert (16) having the given configuration; and
(d) fittingly mounting the radiopaque insert (16) into the cavity formed by the depression (8d) at the septum (8);
wherein the insert (16) provides a readable indicia to a viewer visually and when the septum (8) is viewed under x-ray or computer tomography imaging.

2. The method of claim 1, further comprising the step of:
covering the top surface of the septum (8) where the depression (8d) is formed with a transparent elastomeric layer (18) after the insert (16) has been mounted into the cavity formed by the depression (8d) to insulate the insert (16) from the environment.

3. The method of claim 1, wherein step (c) further comprises the steps of:
configuring a mold (20) to have a mold cavity shaped to form the insert (16, 22a-22g) having the given configuration;
injecting a radiopaque material into the mold cavity (20); and
solidifying the radiopaque material injected into the mold cavity to form the insert (16, 22a-22g) as a radiopaque one piece solid insert.

4. The method of claim 3, wherein the injecting step comprises the step of injecting liquid Barium Sulfate (BaSO4) into the mold cavity.

5. The method of claim 1, further comprising the step of covering the top surface with a silicone layer (18).

6. The method of claim 1, wherein the cavity is formed by the depression (8d) as a continuous cavity of the given configuration from the top surface of the septum (8) to a predetermined depth into the septum.

7. The method of claim 1, wherein step (b) further comprising the step of:
forming the given configuration of the depression (8d) as a readable indicia that is adapted to convey information to a viewer.

8. A port (2) implantable into a patient, comprising:
a housing (10) having a chamber (10b) with an opening and an outlet (10d), the opening having fitted therein a septum (8), the septum (8) sealing the opening of the chamber (10b) to form a reservoir in the housing adapted to store a fluid, a catheter (14) connected to the outlet (10d) to be in fluid communication with the reservoir so that the fluid is conveyable between the reservoir and the patient when the port is implanted into the patient, wherein the septum (8) has a cavity formed at an upper portion (8b) of the septum (8) by a depression (8d) of a given configuration, and **characterized in that** a radiopaque insert (16) is fittingly mounted into the cavity formed by the depression (8d) so that the insert (16) is embedded into the upper portion (8b) of the septum (8), the insert (16) being viewable visually by a viewer, and wherein after the port is implanted in the patient, the insert (16) provides a readable indicia that identifies at least one characteristic of the port under x-ray or computer tomography imaging.

9. The port of claim 8, wherein the septum (8) is a resealable elastomer septum sealingly fitted to the opening;
wherein the outlet (10d) is at a lower portion of the housing (10) to establish a fluid path from the reservoir to outside of the housing;
wherein the septum (8) is adapted to be punctured by a cannula so that fluid may be input to or withdrawn from the reservoir; and
wherein the readable indicia is a visually viewable information bearing indicia that is viewable under x-ray or computer tomography imaging.

10. The port of claim 8 or 9, wherein the insert (16) comprises a molded one piece radiopaque insert (16); and
wherein after the insert (16) is mounted into the depression (8d), the top of the septum (8) is covered by a silicone layer (18) sealingly bonded to the septum (8) to encapsulate the insert (16).

11. The method of claim 1, wherein step c) further comprises the integral molding of the insert (16') with a top layer (38) having a configuration that form fits over the top of a septum base (8a) that includes the upper portion (8b) of the septum (8).

12. The method of claim 11, further comprises the steps of:
impregnating a fluid silicone from which the top layer (38) is formed with a radiopaque material;
injecting the radiopaque material impregnated fluid silicone to a septum top mold (30);
solidifying the injected fluid silicone into a one piece septum top layer (38) with the insert (16) integrally hanging from its bottom surface (38b); and
extracting the one piece radiopaque septum top layer (38) from the septum top mold (30).

13. The method of claim 1 or 11, further comprises the step of:
forming the depression (8d) to have a predetermined depth into the septum (8) from the top surface of the septum (8) so that the insert (16) is adapted to be fully mounted into the depression (8d), the mounted insert (16) providing a readable indicia to convey information to a viewer under x-ray or computer tomography imaging.

14. The port of claim 8, wherein the insert (16) is integrally molded with a top layer (38) to form a one piece septum top where the insert (16') hangs downwardly from a bottom surface (38b) of the top layer (38) for fittingly mounted into the depression (8d) at the septum (8).

15. The port of any of claims 8-10, wherein the cavity formed by the depression (8d) has a curved internal trench surface, and
wherein the insert (16) is molded to have a flat top surface (16a) and an outwardly curved under surface (16b) so that the insert (16) is adapted to fittingly mounted into the cavity.

## Patentansprüche

1. Verfahren zur Herstellung eines markierungstragenden Septums, das folgende Schritte umfasst:
(a) Bereitstellung eines elastomeren Septums (8);
(b) Bilden eines Hohlraums an einem oberen Abschnitt (8b) des Septums (8) durch mindestens eine Vertiefung (8d) einer gegebenen Konfiguration in einer oberen Oberfläche des Septums (8);
und **gekennzeichnet durch** folgende weiteren Schritte:
(c) Formen eines röntgendichten Einsatzes (16) mit der gegebenen Konfiguration; und
(d) Feste Anbringung des röntgendichten Einsatzes (16) in dem durch die Vertiefung (8d) am Septum (8) gebildeten Hohlraum;
wobei der Einsatz (16) einem Betrachter visuell und bei Betrachtung des Septums (8) per Röntgen- oder Computertomographie-Bildgebung eine ablesbare Markierung liefert.

2. Verfahren nach Anspruch 1, ferner folgenden Schritt umfassend:
Abdeckung der oberen Oberfläche des Septums (8), wo die Vertiefung (8d) mit einer transparenten Elastomerschicht (18) ausgebildet ist, nachdem der Einsatz (16) in den durch die Vertiefung (8d) gebildeten Hohlraum montiert wurde, um den Einsatz (16) von der Umgebung zu isolieren.

3. Verfahren nach Anspruch 1, wobei Schritt (c) ferner folgende Schritte umfasst:
Konfigurieren einer Form (20), um einen Formhohlraum zu erhalten, der so geformt ist, dass er den Einsatz (16, 22a-22g) mit der gegebenen Konfiguration bildet;
Einspritzen eines röntgendichten Materials in den Formhohlraum (20); und
Verfestigung des röntgendichten Materials, das in den Formhohlraum eingespritzt wurde, um den Einsatz (16, 22a-22g) als röntgendichten, einteiligen massiven Einsatz zu bilden.

4. Verfahren nach Anspruch 3, wobei der Injektionsschritt den Schritt des Injizierens von flüssigem Bariumsulfat (BaSO4) in den Formhohlraum umfasst.

5. Verfahren nach Anspruch 1, ferner umfassend den Schritt des Bedeckens der Oberseite mit einer Silikonschicht (18).

6. Verfahren nach Anspruch 1, wobei der Hohlraum durch die Vertiefung (8d) als kontinuierlicher Hohlraum der gegebenen Konfiguration von der Oberseite des Septums (8) bis zu einer vorbestimmten Tiefe in das Septum hinein gebildet wird.

7. Verfahren nach Anspruch 1, wobei Schritt (b) ferner den folgenden Schritt umfasst:
Ausbildung der gegebenen Konfiguration der Vertiefung (8d) als lesbare Markierungen, die geeignet sind, Informationen an einen Betrachter zu übermitteln.

8. Ein Anschluss (2), der in einen Patienten implantierbar ist, umfassend:
ein Gehäuse (10) mit einer Kammer (10b) mit einer Öffnung und einem Auslass (10d), wobei in der Öffnung ein Septum (8) angebracht ist, das Septum (8) die Öffnung der Kammer (10b) abdichtet und ein Reservoir im Gehäuse bildet, das geeignet ist, ein Fluid zu speichern, und einen Katheter (14), der mit dem Auslass (10d) verbunden ist, sodass dieser mit dem Fluid im Reservoir in Verbindung steht und das Fluid zwischen dem Reservoir und dem Patienten transportierbar ist, wenn der Port in den Patienten implantiert wird, wobei
das Septum (8) einen Hohlraum aufweist, der an einem oberen Abschnitt (8b) des Septums (8) durch eine Vertiefung (8d) einer gegebenen Konfiguration ausgebildet und **dadurch gekennzeichnet ist, dass** ein röntgendichter Einsatz (16) in dem Hohlraum, der durch die Vertiefung (8d) gebildet wird, passend angebracht ist, sodass der Einsatz (16) in den oberen Abschnitt (8b) des Septums (8) eingebettet ist, wobei der Einsatz (16) von einem Betrachter visuell sichtbar ist, und wobei der Einsatz (16) nach dem Implantieren des Ports in den Patienten eine ablesbare Markierung bietet, die mindestens eine Eigenschaft des Anschlusses bei Röntgen- oder Computertomographie-Bildgebung kennzeichnet.

9. Anschluss nach Anspruch 8, wobei das Septum (8) ein wiederverschließbares Elastomer-Septum ist, das dicht an der Öffnung angebracht ist;
wobei sich der Auslass (10d) an einem unteren Abschnitt des Gehäuses (10) befindet, um einen Fluidweg von dem Reservoir zur Außenseite des Gehäuses herzustellen;
wobei das Septum (8) geeignet ist, durch eine Kanüle punktiert zu werden, sodass Flüssigkeit in das Reservoir eingegeben oder aus dem Reservoir entnommen werden kann; und
wobei die lesbare Markierung eine visuell sichtbare Information mit aus Markierungen ist, die mittels Röntgen- oder Computertomographie-Bildgebung sichtbar ist.

10. Anschluss nach Anspruch 8 oder 9, wobei der Einsatz (16) einen geformten einteiligen röntgendichten Einsatz (16) umfasst; und
wobei nach der Montage des Einsatzes (16) in die Vertiefung (8d) die Oberseite des Septums (8) mit einer Silikonschicht (18) bedeckt wird, die dicht mit dem Septum (8) verbunden ist, um den Einsatz (16) zu verkapseln.

11. Verfahren nach Anspruch 1, wobei Schritt c) ferner das integrale Formen des Einsatzes (16') mit einer Deckschicht (38) umfasst, die eine Konfiguration aufweist, die über die Oberseite einer Septumbasis (8a) passt, die den oberen Teil (8b) des Septums (8) umfasst.

12. Verfahren nach Anspruch 11, ferner umfassend folgende Schritte:
Imprägnieren eines flüssigen Silikons, aus dem die Deckschicht (38) mit einem röntgendichten Material gebildet ist;
Einspritzen des mit röntgendichten Material imprägnierten flüssigen Silikons in eine Septum-Oberform (30);
Verfestigen des eingespritzten flüssigen Silikons in einer einteiligen Septum-Deckschicht (38), wobei der Einsatz (16) in einem Stück von seiner Unterseite (38b) hängt; und
Entnahme der einteiligen röntgendichten Septumdeckschicht (38) aus der Septum-Oberform (30).

13. Verfahren nach Anspruch 1 oder 11, ferner umfassend den folgenden Schritt:
Ausbildung der Vertiefung (8d), damit es eine vorbestimmte Tiefe in das Septum (8) von der oberen Oberfläche des Septums (8) hinein hat, sodass der Einsatz (16) vollständig in die Vertiefung (8d) montiert werden kann, wobei der montierte Einsatz (16) eine ablesbare Markierung liefert, um Informationen an einen Betrachter per Röntgen- oder Computertomographie-Bildgebung zu übermitteln.

14. Anschluss nach Anspruch 8, wobei der Einsatz (16) mit einer oberen Schicht (38) in einem Stück geformt ist, um eine durchgängige Septumoberseite zu bilden, wobei der Einsatz (16') von einer unteren Oberfläche (38b) der oberen Schicht (38) nach unten hängt, um passend in die Vertiefung (8d) im Septum (8) montiert zu werden.

15. Anschluss nach einem der Ansprüche 8-10, wobei der durch die Vertiefung (8d) gebildete Hohlraum eine gekrümmte innere Rinnenoberfläche aufweist, und
wobei der Einsatz (16) so geformt ist, dass er eine flache Oberseite (16a) und eine nach außen gekrümmte Unterseite (16b) aufweist, sodass der Einsatz (16) passend in den Hohlraum montiert werden kann.

## Revendications

1. Procédé de fabrication d'un septum portant une indication, comprenant les étapes :
(a) de fourniture d'un septum élastomère (8) ;
(b) de formation d'une cavité dans la partie supérieure (8b) du septum (8) avec au moins une dépression (8d) d'une configuration donnée dans la superficie supérieure du septum (8) ;
et **caractérisée par** les étapes :
(c) de moulage d'un insert radio-opaque (16) ayant une configuration donnée ; et
(d) de montage, de façon appropriée, de l'insert radio-opaque (16) dans la cavité formée par la dépression (8d) sur le septum (8) ;
où l'insert (16) fournit une indication lisible visuellement à un observateur et lorsque le septum (8) est visualisé sous radiographie ou tomodensitométrie.

2. Procédé selon la revendication 1, comprenant en outre l'étape :
de couverture de la surface supérieure du septum (8) où la dépression (8d) est formée d'une couche d'élastomère transparent (18) après que l'insert (16) ait été monté dans la cavité formée par la dépression (8d) pour isoler l'insert (16) de l'environnement.

3. Procédé selon la revendication 1, dans lequel l'étape (c) comprend en outre les étapes :
de configuration d'un moulage (20) pour obtenir une cavité de moulage pour former l'insert (16, 22a-22g) ayant une configuration donnée ;
d'injection d'un matériau radio-opaque dans la cavité de moulage (20) ; et
de solidification du matériau radio-opaque injecté dans la cavité de moulage pour former l'insert (16, 22a-22g) comme un insert solide monobloc radio-opaque.

4. Procédé selon la revendication 3, dans lequel l'étape d'injection comprend l'étape d'injection de Sulfate de Baryum (BaSO4) liquide dans la cavité de moulage.

5. Procédé selon la revendication 1, comprenant en outre l'étape de recouvrement de la surface supérieure avec une couche de silicone (18).

6. Procédé selon la revendication 1, dans lequel la cavité est formée par la dépression (8d) comme une cavité continue de la configuration donnée depuis la surface supérieure du septum (8) jusqu'à une profondeur prédéterminée dans le septum.

7. Procédé selon la revendication 1, dans lequel l'étape (b) comprend en outre l'étape consistant à :
la formation de la configuration donnée de la dépression (8d) comme une indication lisible adaptée pour apporter une information à un observateur.

8. Chambre (2) implantable chez un patient, comprenant :
une coque (10) ayant un compartiment (10b) avec une ouverture et une sortie (10d), l'ouverture ayant un septum (8) fixé à l'intérieur, le septum (8) scellant l'ouverture du compartiment (10b) pour former un réservoir dans la coque adapté pour stocker un fluide, un cathéter (14) connecté à la sortie (10d) pour être en communication fluide avec le réservoir de sorte que le fluide est transportable entre le réservoir et le patient quand la chambre est implantée chez le patient, dans laquelle le septum (8) a une cavité formée sur une partie plus élevée (8b) du septum (8) via une dépression (8d) d'une configuration donnée, et **caractérisée par le fait qu'**un insert radio-opaque (16) est monté de façon appropriée dans la cavité formée par la dépression (8d) de sorte que l'insert (16) est incrusté dans la partie supérieure (8b) du septum (8), l'insert (16) étant visuellement visible par un observateur, et dans lequel après que la chambre ait été implantée chez le patient, l'insert (16) offre une indication lisible qui identifie au moins une caractéristique de la chambre sous radiographie ou tomodensitométrie.

9. Chambre selon la revendication 8, dans laquelle le septum (8) est un septum élastomère refermable hermétiquement ajusté sur l'ouverture ;
où la sortie (10d) est dans une partie plus basse de la coque (10) pour établir une voie fluide du réservoir vers l'extérieur de la coque ;
où le septum (8) est adapté pour être percé par une canule de sorte que ce fluide puisse être introduit ou retiré du réservoir ; et
où l'indication lisible est une indication portant une information visuellement visible qui est visible sous radiographie ou tomodensitométrie.

10. Chambre selon la revendication 8 ou 9, dans laquelle l'insert (16) comprend un insert radio-opaque monobloc moulé (16) ; et
dans laquelle après que l'insert (16) soit monté dans la dépression (8d), le sommet du septum (8) est recouvert d'une couche de silicone (18) collée hermétiquement sur le septum (8) pour encapsuler l'insert (16).

11. Procédé selon la revendication 1, dans lequel l'étape c) comprend en outre le moulage intégral de l'insert (16') avec une couche supérieure (38) ayant une configuration dont la forme s'ajuste au sommet de la base du septum (8a) qui inclut la partie supérieure (8b) du septum (8).

12. Procédé selon la revendication 11, comprenant en outre les étapes :
d'imprégnation d'une silicone fluide duquel la couche supérieure (38) est formée avec un matériau radio-opaque ;
d'injection du matériau radio-opaque imprégné de silicone fluide dans le moulage du sommet d'un septum (30) ;
de solidification de la silicone fluide injecté en une couche supérieure monobloc radio-opaque du septum (38) avec l'insert (16) intégralement suspendu de sa surface inférieure (38b) ; et
d'extraction de la couche supérieure monobloc radio-opaque du septum (38) du moulage du sommet du septum (30).

13. Procédé selon la revendication 1 ou 11, comprenant en outre l'étape :
de formation de la dépression (8d) de sorte à avoir une profondeur prédéterminée dans le septum (8) à partir de la surface supérieure du septum (8) de sorte que l'insert (16) soit adapté pour être entièrement monté dans la dépression (8d), l'insert monté (16) fournissant une indication lisible pour apporter des informations à un observateur sous radiographie ou tomodensitométrie.

14. Chambre selon la revendication 8, dans laquelle l'insert (16) est intégralement moulé avec une couche supérieure (38) pour former un dessus de septum monobloc où l'insert (16') est suspendu vers le bas d'une surface inférieure (38b) de la couche supérieure (38) pour être montée de façon appropriée dans la dépression (8d) au niveau du septum (8).

15. Chambre selon l'une quelconque des revendications 8 à 10, dans laquelle la cavité formée par la dépression (8d) a une surface du sillon interne arrondie, et
dans laquelle l'insert (16) est moulé pour avoir une surface supérieure plate (16a) et une surface intérieure arrondie vers l'extérieur (16b) de sorte que l'insert (16) soit adapté pour être monté de façon appropriée dans la cavité.
